# EUROPEAN PATENT SPECIFICATION

(11) **EP 3 638 109 B1**
(45) Date of publication and mention of the grant of the patent: **26.10.2022**
(21) Application number: 18817299.3
(22) Date of filing: 07.06.2018
(51) Int. Cl.: G01N 21/552, G01N 21/05, G01N 33/49, G01N 21/03

(54) **METHOD FOR DETERMINING THE CONCENTRATION OF AN ANALYTE IN WHOLE BLOOD**
VERFAHREN ZUR BESTIMMUNG DER KONZENTRATION EINES ANALYTEN IN VOLLBLUT
PROCÉDÉ POUR DÉTERMINER LA CONCENTRATION D'UN ANALYTE DANS LE SANG TOTAL

(30) Priority: 15.06.2017 US 201762520087 P
(43) Date of publication of application: 22.04.2020
(60) Divisional application: 22193163.7
(73) Proprietor: Siemens Healthcare Diagnostics, Inc., Tarrytown, NY 10591 (US)
(72) Inventor: DUBEY, Satish Kumar, Bangalore 562 106 Karnataka (IN); LAL, Ashish Kumar, Bangalore 560 100 (IN); PRABHU, Vishal Manjanath, Bangalore 560 062 Karnataka (IN); LEDDEN, David, Elkhart, Indiana 46514 (US)
(74) Representative: Schweitzer, Klaus
(86) International application number: PCT/US2018/036434
(87) International publication number: WO 2018/231625

(56) References cited:
- EP-A1- 2 793 015
- WO-A1-95/27208
- WO-A1-2016/193066
- WO-A1-2017/200907
- US-A- 5 650 846
- US-A1- 2005 249 633
- US-A1- 2006 204 403
- US-A1- 2008 240 543
- US-A1- 2014 252 214
- US-B1- 6 268 910
- US-B1- 6 366 726

## Description

### FIELD OF TECHNOLOGY

The present disclosure relates to the field of analysis of whole blood and more particularly to the field of determining the concentration of analyte in whole blood.

### BACKGROUND

Hemolysis is a phenomenon wherein the blood cells rupture in whole blood, releasing their content into the blood plasma. This condition may occur due to various reasons such as immune reactions, infections, and medications. Hemolysis may occur within the body of an individual or after the blood has been extracted out of the body. A major cause of hemolysis is the pre-analytical steps of blood sample handling, including collection of the blood sample from the body of an individual. As a result, the individual may have a hemolytic condition, such as sickle cell anemia. During hemolysis, the composition of the blood plasma is altered because of the contents of the blood cells spilling into the blood plasma. If the composition of the blood plasma is altered beyond a certain threshold, the blood sample is flagged for hemolysis. If the composition of the blood plasma is altered beyond a higher threshold, the blood sample may become incapable of further use and therefore has to be rejected. Therefore, the object of the invention is to provide a method to determine concentration of analytes, particularly extracellular hemoglobin, in a whole blood sample.

WO 95/27208 A1 discloses a method of determining one or more dissolved species in a fluid sample comprising transporting the sample through a flow channel which on a wall thereof has a sensing surface capable of binding the species to form an analyte depleted layer extending from the surface, and determining the extent of binding of the species to the sensing surface. The sample may be whole blood and contains suspended or emulsified material, e.g. blood cells, and is transported through the flow channel in a laminar flow such that there is formed a flow core of the suspended or emulsified material and a surrounding liquid layer, e.g. of plasma, near the flow channel wall, which layer is substantially free from suspended or emulsified material. Optical methods may be used to detect mass surface concentration, such as reflection-optical methods, including both internal and external reflection methods, e.g. ellipsometry and evanescent wave spectroscopy (EWS), the latter including surface plasmon resonance spectroscopy (SPRS), Brewster angle refractometry, critical angle refractometry, frustrated total reflection (FTR), evanescent wave ellipsometry, scattered total internal reflection (STIR), optical wave guide sensors, evanescent wave based imaging, such as critical angle resolved imaging, Brewster angle resolved imaging, SPR angle resolved imaging, etc. , as well as methods based on evanescent fluorescence (TIRF) and phosphorescence.

WO 2016/193066 A1 discloses the analysis of whole blood in a flow cell using waveguides which may be arranged inside a fluid channel, wherein sedimentation of the blood cells is used for the analysis in combination with the interaction of the blood sample with the evanescent field on the waveguide surfaces.

EP 2 793 015 A1 discloses the analysis of whole blood flowing in a channel of a flow cell formed of a transparent material thereby forming a cell-free plasma layer at the interface with a channel surface. Measurement light is caused to be incident on the channel surface from an oblique direction at an angle smaller than 90 degrees and reflected light regularly reflected at the boundary surface between the flow cell and the blood is subjected to spectrometry, thereby obtaining information on a plasma component.

WO 2017/200907 A1 is prior art under Article 54(3) EPC and shows the optical determination of an analyte content in a cell free portion of blood without centrifugation or other preliminary steps for separating the cell free portion from whole blood. A channel is configured for containing a flowing blood sample along an optical boundary, so that a cell free layer forms along the boundary of the channel which coincides with the optical boundary. A light beam is directed onto the optical boundary at an angle selected to generate total reflection from the boundary and to generate an evanescent field across the boundary in the cell free layer. A light detector is configured to detect absorption of the light in the evanescent field. The light source and light detector are matched to the wavelength range of an absorption peak of the analyte being detected.

US 2014/252214 A1 discloses a method for analyzing a fluid by evanescence field spectroscopy. The device includes a waveguide, a source of electromagnetic radiation coupled to the waveguide on the entry side, and a detector coupled to the waveguide on the exit side for detecting electromagnetic radiation, wherein the waveguide is arranged in contact with the fluid in at least certain sections between its entry side and its exit side. An electrode arrangement is also provided, which is designed to generate an inhomogeneous electric field in the direct environment of the waveguide, said field exerting a dielectrophoretic force on polarisable particles in the fluid, which moves these particles towards or away from the waveguide. The waveguide may be an optical fiber arranged within a tube-shaped flow cell concentrically surrounding the optical fiber. The optical fiber may be free from the outer cladding, at least in the section where it comes in contact with the fluid, so that the fluid can contact the surface of the core.

### SUMMARY

The present invention is defined in claim 1.

A method for determining the concentration of an analyte in whole blood sample is disclosed. In one aspect of the invention, the method includes generating a plasma layer in the whole blood sample. The method also includes exposing the plasma layer to light. Furthermore, the method includes capturing light reflected from the plasma layer. Additionally, the method also includes analyzing the reflected light to determine the concentration of the analyte.

In the present invention, which is defined in claim 1, a device for determining the concentration of an analyte in whole blood sample is used, which includes a microfluidic channel configured to carry whole blood; a light source configured to direct light into an optical fiber, which is placed inside the microfluidic channel; and a measuring unit. The measuring unit is configured to capture light reflected multiple times between the surfaces of the optical fiber. Furthermore, the measuring unit is configured to compute the concentration of the analyte in the whole blood.

This summary is provided to introduce a selection of concepts in a simplified form that are further described below in the following description.

### BRIEF DESCRIPTION OF THE DRAWINGS

The following description refers to the accompanying drawings, in which:
Figure 1 illustrates a flowchart of an exemplary method of determining the concentration of an analyte in whole blood.
Figure 2A illustrates a side view of an illustrative device which can be used to determine the concentration of an analyte in plasma.
Figure 2B illustrates a vertical cross sectional view of the illustrative device which can be used to determine the concentration of an analyte in plasma.
Figure 3A illustrates a side view of an embodiment of an illustrative device which can be used to determine the concentration of an analyte in plasma.
Figure 3B illustrates a schematic representation of a cross sectional view of the illustrative device which can be used to determine the concentration of analyte in plasma.

### DETAILED DESCRIPTION

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments.

Optical detection of hemolysis in whole blood can be challenging because of high interference from blood cells, specifically red blood cells (RBCs). Separating blood plasma from whole blood in order to detect hemolysis is time consuming and arduous. Therefore, there exists a need for a method that can detect hemolysis which does not require separation of blood plasma from whole blood, which is faster and cost efficient.

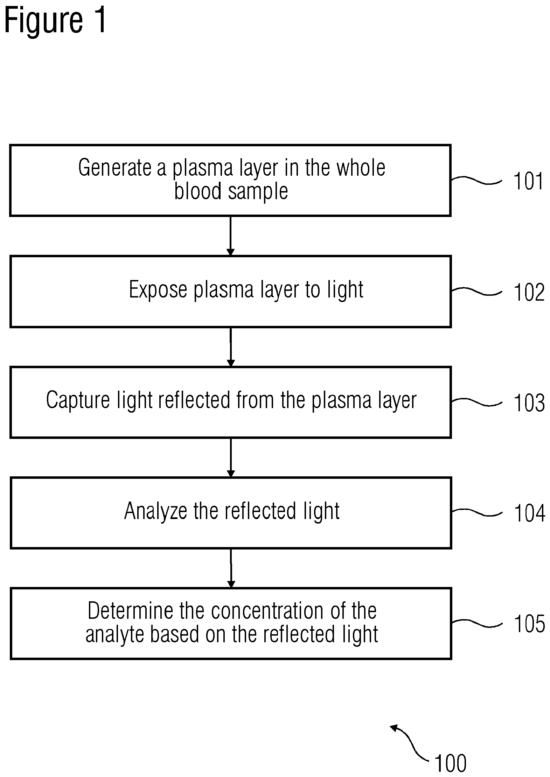

Figure 1 illustrates a flowchart of an exemplary method 100 of determining the concentration of an analyte present in whole blood. The method 100 includes step 101 of generating a plasma layer in the whole blood sample. The plasma layer in the whole blood sample can be generated by passing the sample through a fluidic channel, for example, a microfluidic channel. In examples, which do not form part of the present invention, the fluidic channel may be made of a transparent medium, for example, glass and includes an outer surface and an inner surface. When whole blood flows through a channel with a narrow diameter, the blood cells migrate to the center of the channel, thereby generating a layer of plasma at the walls of the channel. This phenomenon is termed as 'Fahraeus effect'. Fahraeus effect results in decrease in the average concentration of red blood cells when the diameter of the channel through which the blood flows decreases. During hemolysis, the red blood cells rupture, thereby resulting in spilling of the contents of the red blood cells, including analytes such as hemoglobin into the plasma.

By utilizing the Fahraeus effect, the concentration of the red blood cells can be decreased along the walls of the microfluidic channel. Therefore, the plasma layer generated along the walls of the microfluidic channel is devoid of red blood cells. Thus, the concentration of analytes, such as hemoglobin, in the plasma layer can be effectively determined without interference from the blood cells.

At step 102, the generated plasma layer is exposed to light. The plasma layer is irradiated with light of a wavelength in the range between 400-750 nm at an angle greater than the total internal reflection critical angle. The total internal reflection critical angle is the angle of incidence for which the light totally reflects from an interface. The incident light passes through the medium of the microfluidic channel and interacts with the plasma layer generated at the walls of the channel. An index matching substance may be used along with the microfluidic channel so as to ensure that the irradiated light is reflected off the plasma layer and not the surface of the microfluidic channel. Examples of index matching substances include fluids and solids. Examples of index matching fluids include, but are not limited to, paraffin, glycerin, and sugar solution. Examples of index matching solids include, but are not limited to, glass. The microfluidic channel may also be pasted on to another piece of glass. The refractive index of the adhesive used between the microfluidic channel and the piece of glass should be the same as the refractive index of glass. Alternatively, the microfluidic channel may also be etched on a glass surface using techniques well known in the state of the art.

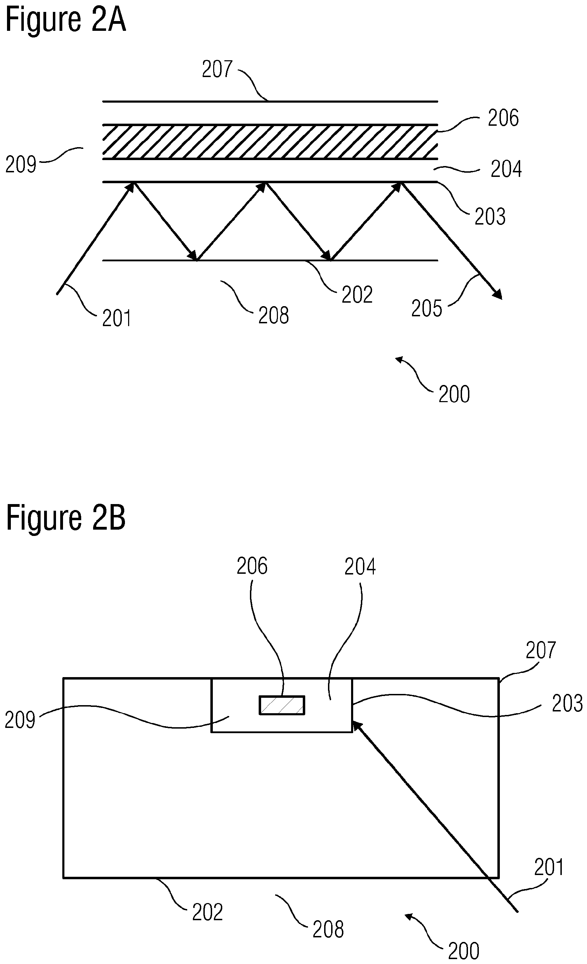

The irradiated light may be reflected multiple times by the plasma layer, as depicted in Figure 2A. Figure 2A illustrates a side view of an illustrative device 200 which can be used to determine the concentration of an analyte in plasma by a method, which is not in accordance with the present invention. The device is a fluidic device, such as a microfluidic device. In the example, a channel 209 is etched on a first layer of transparent material 208. The channel 209 may be, for example, a microfluidic channel. The first layer of transparent material 208 includes an outer surface 202 and an inner surface 203. The microfluidic channel 209 is defined by the inner surface 203 of the transparent material 208. A second layer of transparent material 207 is placed over the first layer of transparent material 208. The layers of transparent material 207, 208 may be made of index matching substances, for example, glass. The microfluidic channel 209 contains a whole blood sample. Using the Fahraeus Effect, the red blood cells 206 migrate to the center of the microfluidic channel 209, thereby generating a cell-free plasma layer 204 along the inner surface 203 of the microfluidic channel 209. Figure 2B illustrates a vertical cross sectional view of the illustrative device 200. The red blood cells 206 gather at the center of the microfluidic channel 209 using Fahraeus Effect. A layer of plasma 204 is generated around the red blood cells. The irradiated light 201 enters the glass surface and interacts with the plasma layer 204 at the inner surface 203 of the micro fluidic channel 209. On hitting the inner surface 203 of the microfluidic channel 209, the light can be reflected multiple times between the inner and outer surfaces of the microfluidic channel 209. The critical angles for total internal reflection are different for the air-glass interface and glass-plasma interface. The chosen incident angle in Figs. 2A and 2B is greater than the critical angles for the air-glass interface and glass-plasma interface to ensure multiple reflections. At step 103, the reflected light 205 is captured. The reflected light 205 may be captured using a spectrophotometer. At step 104, the captured reflected light 205 is analyzed. The reflected light 205 may vary according to the concentration of the analyte in the separated plasma layer 204. At step 105, the concentration of the analyte is determined based on the reflected light 205.

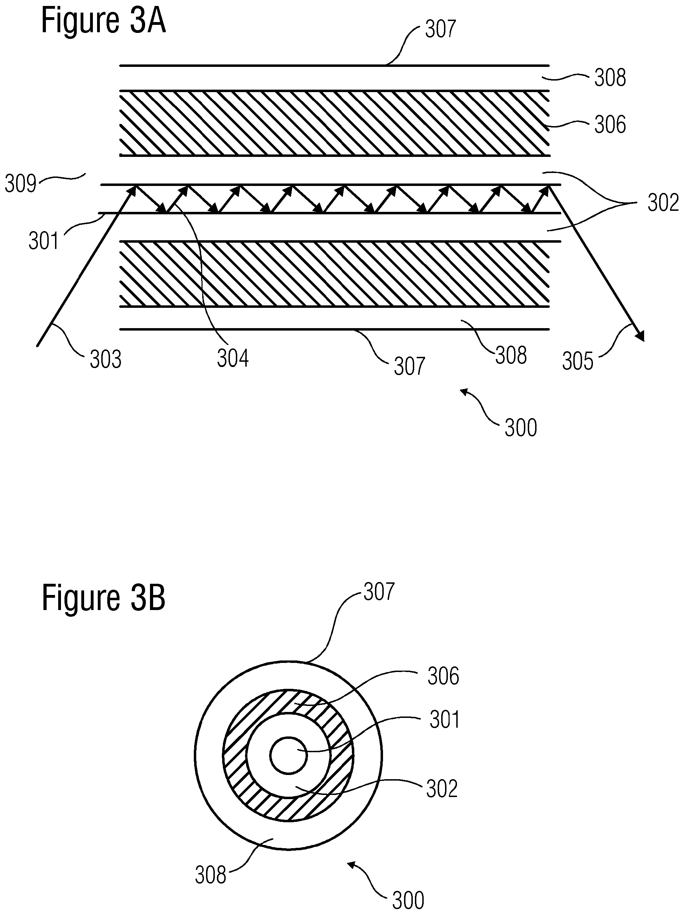

Figure 3A illustrates a side view of an embodiment of an illustrative device 300 which is used in the present invention to determine the concentration of an analyte in plasma. In the embodiment, the device 300 includes an optical fiber 301 that is located within the channel 309. The channel 309 is a microfluidic channel. In illustrative embodiments, the optical fiber 301 may be located in or towards the center of the channel 309 or adjacent to the inner surface 307 of the channel 309. The whole blood sample flows through the microfluidic channel 309 at a constant flow rate. The rate at which the whole blood flows through the microfluidic channel 309 is within a range at which Fahraeus Effect can be effectively achieved. The optical fiber 301 may be a thin, transparent fiber of glass or plastic and is without cladding. Figure 3B illustrates a vertical cross sectional view of the device 300, having a channel 309 with the optical fiber 301 located in the center. The channel 309 is defined by the inner surface 307 of the device 300. The optical fiber 301 is placed parallel to the central axis of the microfluidic channel 309. The central axis of the microfluidic channel 309 is parallel to the flow path of the whole blood in the microfluidic channel 309. According to the present invention, the optical fiber 301 is placed parallel to the central axis such that the optical fiber 301 is not in contact with the inner surface 307 of the microfluidic channel 309. Due to the placement of the optical fiber 301 inside the microfluidic channel 309, a layer of cell-free plasma 302 is generated using Fahraeus Effect, around the optical fiber 301. An additional layer of plasma 308 is also formed along the walls of the microfluidic channel 309 using Fahraeus Effect. In between cell-free plasma layers 302 and 308, a layer of red blood cells 306 is formed. In the embodiment illustrated in Figs. 3A and 3B, this layer of red blood cells 306 extends along, and encircles, the optical fiber. In other arrangements, which are not in accordance with the present invention, the optical fiber 301 may be placed in contact with the inner surface of the microfluidic channel 309. When light 303 is irradiated into the optical fiber 301, the light 303 interacts with the separated layer of plasma and gets reflected. Within the optical fiber 301, the light 303 may be reflected multiple times 304 between the surfaces of the optical fiber 301. The phenomenon of multiple reflections 304 of the irradiated light 303 allows for signal amplification and therefore enables accurate determination of the concentration of the analyte present in the whole blood sample. Therefore, the reflected light 305 is further captured by the spectrophotometer and a change in the reflected light 305 is detected. Based on the reflected light 305, the concentration of the analyte in the whole blood sample is determined.

The method 100 enables measurement of hemolysis in the whole blood sample in a microfluidic environment. Therefore, the sample volume requirements are low. Furthermore, as no additional reagents are required for the determination of the concentration of the analyte, the method is cost effective. The whole blood sample may also be retrieved for further analysis or downstream processing once the process of determination of the concentration of the analyte is completed.

## Claims

1. . A method of determining the concentration of an analyte in a whole blood sample, the method comprising:
flowing a whole blood sample through a microfluidic channel (309) defined by an inner surface (307) and wherein an optical fiber (301) is used inside the microfluidic channel (309), wherein the whole blood surrounds the optical fiber (301) as it flows through the microfluidic channel (309), and wherein the optical fiber (301) is placed parallel to the central axis of the microfluidic channel (309) such that the optical fiber (301) is not in contact with the inner surface (307) of the microfluidic channel (309), generating a cell-free plasma layer (302) around the optical fiber (301), and a cell-free plasma layer (308) formed along the inner surface (307) of the microfluidic channel (309), using Fahraeus Effect, and a layer of red blood cells (306) in between the cell-free plasma layers (302, 308);
irradiating light into the optical fiber (301), thereby directing light (301) at the plasma layer (302);
capturing light (305) reflected off of the plasma layer (302); and
analyzing the reflected light (305) to determine the concentration of the analyte.

2. . The method of claim 1, wherein light (301) is directed at the inner surface of the optical fiber (301), thereby creating multiple reflections of the exposed light.

3. . The method of claim 1, wherein the reflected light (305) is captured using a spectrophotometer.

4. . The method of claim 1, wherein the analyte is hemoglobin.

## Patentansprüche

1. Verfahren zur Bestimmung der Konzentration eines Analyten in einer Vollblutprobe, wobei das Verfahren umfasst:
Fließenlassen einer Vollblutprobe durch einen Mikrofluidkanal (309), der durch eine Innenfläche (307) definiert wird und wobei eine optische Faser (301) innerhalb des Mikrofluidkanals (309) verwendet wird, wobei das Vollblut die optische Faser (301) umgibt, wenn es durch den Mikrofluidkanal (309) fließt, und wobei die optische Faser (301) parallel zur Mittelachse des Mikrofluidkanals (309) angeordnet ist, derart dass die optische Faser (301) nicht mit der Innenfläche (307) des Mikrofluidkanals (309) in Kontakt ist,
Erzeugen einer zellfreien Plasmaschicht (302) um die optische Faser (301) und einer zellfreien Plasmaschicht (308), die entlang der Innenfläche (307) des Mikrofluidkanals (309) gebildet wird, unter Verwendung des Fåhræsus-Effekts und einer Schicht von roten Blutzellen (306) zwischen den zellfreien Plasmaschichten (302, 308);
Strahlen von Licht in die optische Faser (301), um dadurch Licht (301) auf die Plasmaschicht (302) zu richten;
Erfassen von Licht (305), das von der Plasmaschicht (302) reflektiert wird; und
Analysieren des reflektierten Lichts (305), um die Konzentration des Analyten zu bestimmen.

2. Verfahren nach Anspruch 1, wobei Licht (301) auf die Innenfläche der optischen Faser (301) gerichtet wird, um dadurch mehrere Reflexionen des Bestrahlungslichts zu erzeugen.

3. Verfahren nach Anspruch 1, wobei das reflektierte Licht (305) unter Verwendung eines Spektrofotometers erfasst wird.

4. Verfahren nach Anspruch 1, wobei der Analyt Hämoglobin ist.

## Revendications

1. Procédé de détermination de la concentration d'un analyte dans un échantillon de sang entier, le procédé comprenant :
l'écoulement d'un échantillon de sang entier à travers un canal microfluidique (309) défini par une surface interne (307) et où une fibre optique (301) est utilisée à l'intérieur du canal microfluidique (309), où le sang entier entoure la fibre optique (301) lorsqu'il s'écoule à travers le canal microfluidique (309), et où la fibre optique (301) est placée parallèlement à l'axe central du canal microfluidique (309) de sorte que la fibre optique (301) n'est pas en contact avec la surface interne (307) du canal microfluidique (309),
la génération d'une couche de plasma sans cellules (302) autour de la fibre optique (301), et d'une couche de plasma sans cellules (308) formée le long de la surface interne (307) du canal microfluidique (309), en utilisant l'effet Fahraeus, et d'une couche de globules rouges (306) entre les couches de plasma sans cellules (302, 308) ;
l'irradiation de la lumière dans la fibre optique (301), dirigeant ainsi la lumière (301) vers la couche de plasma (302) ;
la capture de la lumière (305) réfléchie par la couche de plasma (302) ; et
l'analyse de la lumière réfléchie (305) pour déterminer la concentration de l'analyte.

2. Procédé de la revendication 1, dans lequel la lumière (301) est dirigée vers la surface interne de la fibre optique (301), créant ainsi des réflexions multiples de la lumière exposée.

3. Procédé selon la revendication 1, dans lequel la lumière réfléchie (305) est capturée en utilisant un spectrophotomètre.

4. Procédé selon la revendication 1, dans lequel l'analyte est l'hémoglobine.
